# EUROPÄISCHE PATENTANMELDUNG

(11) **EP 1 189 057 A2**
(43) Veröffentlichungstag der Anmeldung: **20.03.2002**
(21) Anmeldenummer: 01119910.6
(22) Anmeldetag: 17.08.2001
(51) Int. Cl.: G01N 27/447, G01N 1/28

(54) **Verfahren und Vorrichtung zur Erfassung von Merkmalen durch Analyse einer Probe menschlicher oder tierischer Körperstoffe**

(30) Priorität: 30.08.2000 DE 10042847
(71) Anmelder: Novel Science International GmbH, 37073 Göttingen (DE)
(72) Erfinder: Thürk, Marcel, Dr., 37120 Bovenden (DE); Goetz, Rüdiger, Dr., 37073 Göttingen (DE); Wichard, Jörg, Dr., 37085 Göttingen (DE); Merkwirth, Christian, Dr., 37073 Göttingen (DE); Evert, Fabian, Dr., 37124 Rosdorf (DE); Kamphausen, Stefan, 37130 Reinhausen (DE); Brunzema, Claus, 37085 Göttingen (DE); Wiesenfeldt, Martin, Dr., 65843 Sulzbach (DE)
(74) Vertreter: Braun, Dieter, Dipl.-Ing.

(57) **Zusammenfassung**

Die Erfindung betrifft ein Verfahren und eine Vorrichtung (6) zur Erfassung von Merkmalen, die durch Analyse einer Probe (1) menschlicher oder tierischer Körperstoffe, insbesondere Blut oder Gewebe, gewonnen wird. Hierzu wird zunächst ein an sich bekanntes Trennverfahren eingesetzt, um die Probe (1) auf der Unterlage (7) zu verteilen. Anschließend wird die Probe (1) in einzelne Abschnitte (8) unterteilt und bei der nachfolgenden Analyse die Probe (1) auf der Unterlage (7) in einer unveränderten Position untersucht. Die Auswahl der jeweils geeigneten Verfahren zur Analyse der Probe sowie der zu untersuchenden Abschnitte (8) wird in Abhängigkeit der Merkmalserfassung vorhergehender Abschnitte (8) bestimmt. Dadurch kann die für die abschließende Diagnose entscheidende Präzisierung der Merkmalserfassung während der Durchführung vorgenommen werden, so daß das Ergebnis erheblich schneller erreicht wird. Weiterhin eignet sich die Vorrichtung (6) durch ihre geringen äußeren Abmessungen insbesondere auch für den mobilen Einsatz vor Ort.

## Beschreibung

Die Erfindung betrifft ein Verfahren zur Erfassung von Merkmalen durch Analyse einer Probe menschlicher oder tierischer Körperstoffe, insbesondere Blut oder Gewebe, bei dem ein Trennverfahren auf einer Unterlage zur Vorbereitung einer nachfolgenden Analyse der Ausgangsprodukte des Trennverfahrens eingesetzt wird. Weiterhin betrifft die Erfindung eine Vorrichtung zur Durchführung des Verfahrens.

In der Praxis wird ein solches Verfahren zur Merkmalsdetektion von Blut-, Gewebe- oder anderer menschlicher oder tierischer Materialproben in der Medizintechnik eingesetzt. Dabei wird als Trennverfahren insbesondere eine flächenhafte Elektrophorese zur Trennung von Zellbestandteilen eingesetzt. Diese werden dabei auf der Unterlage verteilt und zur Analyse schrittweise entnommen.

Eine solche Vorrichtung ist ferner beispielsweise durch die US 60 65 754 bekannt, bei der einzelne Proben mittels eines Roboters aus der Phoresefläche entnommen und der nachfolgenden Analyse zugeführt werden.

Weiterhin offenbart die US 52 45 185 bereits eine andere Vorrichtung, bei der mittels Laserionisation die Probe aus der Phoresefläche der Nachuntersuchung zugeführt werden kann.

Bei beiden Verfahren hat sich in der Praxis der erhebliche Aufwand als nachteilig erwiesen, der erforderlich ist, um die mit dem Trennverfahren auf der Unterlage verteilte Probe zu entnehmen und der nachfolgenden Analyse zuzuführen. Hierzu sind insbesondere Handhabungshilfen erforderlich, die im wesentlichen ausschließlich unter Laborbedingungen bzw. im stationären Umfeld erreicht werden können. Die bekannten Verfahren eignen sich daher nicht für den mobilen Einsatz, beispielsweise am Unfallort. Dadurch wird in der Praxis wertvolle Zeit verloren, die insbesondere für eine zuverlässige Diagnose in Notfällen oftmals nicht zur Verfügung steht.

Der Erfindung liegt daher die Aufgabe zugrunde, ein Verfahren zur Erfassung von Merkmalen zu schaffen, welches durch die einfache Handhabung neben der stationären Anwendung insbesondere auch den Einsatz vor Ort gestattet. Weiterhin soll eine Vorrichtung zur Durchführung des Verfahrens geschaffen werden.

Die erstgenannte Aufgabe wird erfindungsgemäß mit einem Verfahren gemäß den Merkmalen des Patentanspruchs 1 gelöst.

Die weitere Ausgestaltung des erfindungsgemäßen Verfahrens ist den Unteransprüchen zu entnehmen.

Erfindungsgemäß ist also ein Verfahren vorgesehen, bei dem die Probe in einzelne Abschnitte unterteilt und eine gezielte Analyse der Körperstoffe verschiedener Abschnitte auf der Unterlage durchgeführt wird. Dadurch, daß die Analyse der Körperstoffe unmittelbar auf der Unterlage erfolgt, kann der Aufwand, der nach dem Stand der Technik zur Behandlung der Probe erforderlich ist, entfallen. Daher eignet sich das erfindungsgemäße Verfahren in besonderer Weise auch für den mobilen Einsatz am Unfallort, wobei insbesondere mechanische Hilfen, wie beispielsweise Roboterarme, verzichtbar sind. Dabei läßt sich zugleich die Genauigkeit bei der Erfassung der Merkmale wesentlich steigern, da die Probe zur Analyse an Ort und Stelle auf der Unterlage verbleiben kann und somit nach der Durchführung des Trennverfahrens im wesentlichen keine weiteren äußeren Einflüsse erfährt. Messwertabweichungen, verursacht durch die Probenentnahmen, sind somit ausgeschlossen.

Eine besonders vorteilhafte Ausgestaltung der vorliegenden Erfindung wird dadurch erreicht, daß die Analyse eines nachfolgenden Abschnittes durch die erfaßten Merkmale des vorangegangenen Abschnittes der Probe bestimmt wird. Hierdurch werden aus der vorangegangenen Probe im Rahmen der signifikanten Größen und der daraus ableitbaren Merkmale bereits die Voraussetzungen für die Analyse des nachfolgenden Abschnittes geschaffen, um somit schneller und mit größerer Zuverlässigkeit zum Ergebnis zu kommen. Im Gegensatz zu den nach dem Stand der Technik bekannten Verfahren, bei denen die Abfolgen der Analyse verschiedener Abschnitte im vorhinein und um wesentlichen unveränderbar festgelegt sind, wird hierbei eine dynamische Anpassung der Abfolge erreicht, bei der die Einzelergebnisse der verschiedenen Abschnitte in Beziehung zueinander gesetzt werden. Auf diese Weise läßt sich die Datenmenge reduzieren, indem erkennbare logische Zusammenhänge zwischen den Analyseergebnissen verschiedener Abschnitte zur Auswertung hinzugezogen werden und damit bereits als absehbare unbrauchbare Merkmale im folgenden nicht weiter berücksichtigt werden. Dadurch sinkt die erforderliche Zeit zur Durchführung des Verfahrens bzw. die Analysezeit.

Hierbei ist eine Ausgestaltung der Erfindung dann besonders vorteilhaft, wenn aufgrund der erfaßten Merkmale des vorangegangenen Abschnittes das Analyseverfahren bestimmt wird. Hierdurch kann auf das nach dem Trennverfahren durchzuführende Analyseverfahren bereits nach der ersten Merkmalserfassung Einfluß genommen werden. Daher wird ein fallweise ungeeignetes Verfahren als solches erkannt und aufgrund bekannter Expertensysteme ein geeignetes Verfahren ermittelt. Die mangelhafte Erfassung von Merkmalen aufgrund eines ungeeigneten Verfahrens kann dadurch rechtzeitig erkannt und daraus unmittelbar ein geeignetes Verfahren bestimmt werden, bevor eine vorgegebene Messreihe abgeschlossen ist.

Hierbei ist es auch besonders günstig, wenn aufgrund des Ergebnisses der Analyse des vorangegangenen Abschnittes der nachfolgend zu analysierende Abschnitt ausgewählt wird. Hierdurch werden solche Abschnitte, deren Anteil der Probe lediglich in geringem Umfang verwertbar ist, aufgrund vorhergehender Abschnitte erfaßt und dadurch gezielt die Messwerterfassung auf entsprechende nachfolgende Abschnitte gerichtet. Dabei können sowohl die benachbarten Abschnitte als auch die abgewandten Abschnitte für die Messwerterfassung von erhöhter Bedeutung sein, was sich jeweils in Einzelfall aus der Art und der Größe der Merkmale ergibt.

Eine weitere, ebenfalls besonders praxisnahe Abwandlung des erfindungsgemäßen Verfahrens wird dadurch erreicht, daß durch die Analyse zunächst eine Grobklassifikation vorgenommen wird, um so eine Eingrenzung der nachfolgend zu erfassenden Merkmale zu erreichen. Hierdurch wird eine Selektion der Merkmale aufgrund einer übergeordneten Klassifikation vorgenommen, um so die Erfassung unnötiger Merkmale zu vermeiden. Hierdurch kann der Rechenaufwand sowie der damit verbundene Zeitaufwand wesentlich vermindert werden.

Ebenfalls besonders günstig ist es, wenn mehrere Analyseverfahren nacheinander durchgeführt werden, wobei die Auswahl oder die Reihenfolge durch das Ergebnis zumindest eines vorangegangenen Analyseverfahrens bestimmt wird. Hierdurch wird das jeweils geeignete Analyseverfahren den vorhergehenden Ergebnissen angepaßt, um so insbesondere zur einer schnellen Präzisierung und damit zu einer frühzeitigen Diagnose zu kommen. Dabei ist ein Rückgriff auf bereits untersuchte Abschnitte bei Zweckdienlichkeit ebenso möglich, wie eine Bestimmung der Merkmale mit einem weiteren Analyseverfahren zu Kontrollzwecken.

Eine besonders zweckmäßige Weiterbildung der Erfindung wird auch erreicht, indem die Abfolge der Analyse der verschiedenen Abschnitte zusätzlich als Grundlage der Messerfassung hinzugezogen wird. Hierdurch können bereits aus dem nachvollziehbaren Ablauf der Analyse für die anschließende Diagnose wichtige Hinweise entnommen werde, die für die abschließende Beurteilung der Ergebnisse von entscheidender Bedeutung sein können.

Die zweitgenannte Aufgabe, eine Vorrichtung zur Erfassung von Merkmalen durch Analyse einer Probe menschlicher oder tierischer Körperstoffe, insbesondere Blut oder Gewebe, mit einer die Körperstoffe tragenden Unterlage zur Durchführung des Verfahrens zu schaffen, wird erfindungsgemäß dadurch gelöst, daß die Vorrichtung zur ortsaufgelösten Erfassung von Meßwerten der auf der Unterlage aufliegenden Körperstoffe ausgeführt ist. Hierdurch kann die Probe zur Analyse auf der Unterlage verbleiben, so daß einerseits ein zusätzlicher Aufwand zur Handhabung entfällt, andererseits zugleich auch nachteilige und das Ergebnis beeinflussende Umgebungseinflüsse vermindert oder ausgeschlossen werden. Dabei können die Abmessungen der Vorrichtung derart vermindert werden, daß hierdurch eine problemlose Mitführbarkeit für einen mobilen Einsatz vor Ort gewährleistet wird. Die Vorrichtung erfordert ferner einen verminderten Aufwand bei der Herstellung.

Besonders vorteilhaft ist auch eine Ausführungsform der erfindungsgemäßen Vorrichtung, bei der die Vorrichtung zur optischen Merkmalserfassung mit einer Lichtquelle und einem optischen Sensor ausgeführt ist. Hierdurch kann die Analyse ohne einen unmittelbaren Kontakt mit der Probe durchgeführt werden, so daß Fremdeinflüsse sehr gering gehalten werden können. Dabei kommen für das Licht verschiedene Wellenlängenbereiche vom infraroten über den sichtbaren, bis zum ultravioletten Spektralbereich in Frage. Der von der Lichtquelle abgestrahlte Lichtstrahl trifft nach einer geeigneten Fokussierung auf die Probe, die dabei insbesondere durchleuchtet wird. Die Abweichung der Kenngrößen des Lichtes wird dabei mittels des optischen Sensors erfaßt.

Die Lichtquelle kann hierzu beweglich angeordnet sein. Besonders zweckmäßig ist es hingegen, wenn die Vorrichtung zur Analyse verschiedener Abschnitte der Probe eine steuerbare Lichtablenkung aufweist. Hierdurch kann die Lichtquelle insbesondere ortsfest fixiert werden, wobei gezielte Lichteinwirkung auf bestimmte Abschnitte der Probe durch eine Lichtumlenkung, beispielsweise mittels eines Spiegels und durch Fokussierung, erreicht wird.

Hierzu eignet sich eine Abwandlung in besonders vorteilhafter Weise, bei der die Unterlage transmissiv ist. Der optische Sensor zur Erfassung des durch die Probe hindurchtretenden Lichtes kann dadurch unmittelbar an einer der Probe abgewandten Seite der Unterlage angeordnet werden, wodurch sich eine geringe Störanfälligkeit sowie ein geringer Platzbedarf ergibt.

Eine andere besonders vorteilhafte Abwandlung wird dadurch erreicht, daß die Vorrichtung zur Analyse verschiedener Abschnitte der Probe mit einer relativ beweglichen Unterlage ausgestattet ist. Hierbei kann insbesondere die Unterlage gegenüber der übrigen Bauelementen der Vorrichtung relativ beweglich angeordnet sein, um so beliebige Abschnitte der auf der Unterlage aufliegenden Probe einer eingehenden Analyse zu unterziehen.

Eine weitere besonders zweckmäßige Ausführungsform wird auch dadurch geschaffen, daß die Vorrichtung eine Ultraschallquelle und einen Ultraschallsensor aufweist. Hierdurch können neben optischen Analyseverfahren auch Ultraschalluntersuchungen durchgeführt werden, wodurch sich die Zuverlässigkeit der daraus erstellten Diagnose weiter verbessern läßt.

Besonders praxisnah ist es auch, wenn die Vorrichtung zusätzlich einen dem jeweiligen Abschnitt der Unterlage zuordnenbaren Sensor aufweist. Dieser Sensor kann dem jeweils zu untersuchenden Abschnitt entweder unveränderlich oder auch bedarfsweise zugeordnet werden, um lediglich in dem gewünschten Abschnitt die erforderliche Messung durchzuführen. Einfluß benachbarter Abschnitte kann somit ausgeschlossen werden.

Es ist weiterhin besonders zweckmäßig, wenn der zusätzliche Sensor zur Erfassung der elektrischen Leitfähigkeit oder des PH-Wertes bestimmt ist, um so weitere Merkmale zu erfassen, durch welche die Zuverlässigkeit der Analyse weiter erhöht werden kann.

Die Erfindung läßt verschiedene Ausführungsformen zu. Zur weiteren Verdeutlichung ihres Grundprinzips ist eine davon in der Zeichnung dargestellt und wird nachfolgend beschrieben. Diese zeigt in
- Fig.1: ein Ablaufdiagramm des erfindungsgemäßen Verfahrens;
- Fig.2: eine Prinzipskizze einer Vorrichtung zur Durchführung des in Figur 1 dargestellten Verfahrens in einer Seitenansicht.

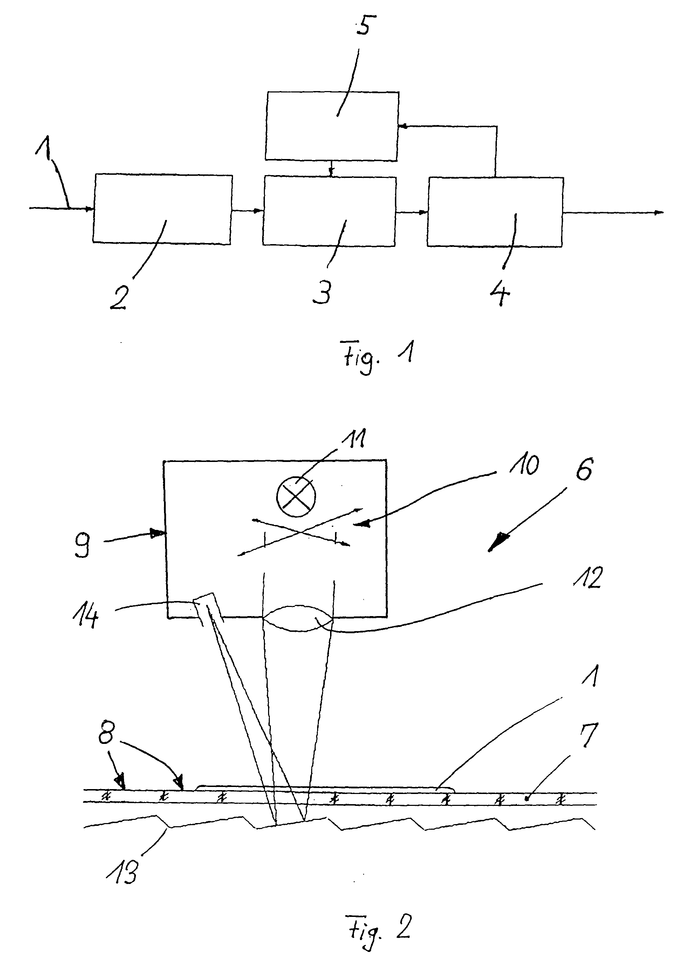

Figur 1 zeigt ein Ablaufdiagramm des erfindungsgemäßen Verfahrens. Eine in Figur 2 näher dargestellte Probe 1 wird dabei der Analyse zugeführt, die in einem ersten Verfahrensschritt aus einem elektrophoretischen Trennverfahren 2 besteht. Ein nachfolgendes, mittels der in Figur 2 im einzelnen dargestellten Vorrichtung 6 durchführbares Analyseverfahren 3 liefert die notwendigen Merkmale für eine Grobklassifikation der Probe 1. So könnte sich aus dieser ersten Analyse beispielsweise der Verdacht auf mehrere Fehlfunktionen im Körper des Patienten ergeben, die zu dem sich aus der Merkmalsanalyse ergebenden Probenbild führen. Diese Merkmalsanalyse wird von einer Auswerteeinheit 4 übernommen und einer Steuereinheit 5 zugeführt. Durch diese wird das in den nachfolgenden Analyseschritten durchzuführende Verfahren entsprechend der bereits erfaßten Merkmale modifiziert. Diese Modifikation kann mechanischer, elektrischer oder logischer Natur sein. Ist beispielsweise in der ersten Grobklassifikation die Zusatzinformation durch ein IR-Spektrum im nahen Infrarotbereich gegeben, so könnte diese Modifikation beispielsweise darin bestehen, daß die Auflösung in einem bestimmten Wellenlängenfenster heraufgesetzt wird, oder daß ein anderer Wellenlängenbereich erfaßt oder die Impulsantwort auf einen Ultraschallpuls herangezogen wird. Diese Rekonfiguration wird iterativ so lange vollzogen, bis die Diagnose für den zu prüfenden Diagnosepunkt oder die zu prüfenden Diagnosepunkte abgeschlossen ist. Anschließend können die noch nicht angewendeten Analyseverfahren durchgeführt werden.

Figur 2 zeigt eine Prinzipskizze der Vorrichtung 6, mit deren Hilfe das Analyseverfahren durchgeführt werden kann. Auf einer flächigen und als Elektrophoreseplatte ausgeführten Unterlage 7, die in einzelne Abschnitte 8 unterteilt ist, liegt die Probe 1 als Ausgangsprodukt des vorangegangenen Trennverfahrens auf. Zur optischen Merkmalserfassung hat die Vorrichtung 6 eine optische Einheit 9, die bei dem dargestellten Ausführungsbeispiel mittels einer Verschiebeeinheit 10 relativ zu der Unterlage 7 beweglich ist. Ein von einer Lichtquelle 11 abgestrahlter Lichtstrahl wird dabei zunächst durch eine Linse 12 fokussiert und trifft anschließend auf die Probe 1. Die Probe 1 wird dabei zumindest teilweise durchleuchtet, so daß der Lichtstrahl im folgenden zunächst durch die hierzu transmissive Unterlage 7 hindurchtritt und an einer auf einer der optischen Einheit 9 abgewandten Seite der Unterlage 7 auf eine Reflexionseinheit 13 trifft, durch die der Lichtstrahl erneut durch die Probe 1 und schließlich auf einen als Photodetektor ausgeführten optischen Sensor 14 gelenkt wird. Dabei kommen für das Licht verschiedene Wellenlängenbereiche vom fernen infraroten bis hin zum ultravioletten Spektralbereich in Frage.

## Patentansprüche

1. Verfahren zur Erfassung von Merkmalen durch Analyse einer Probe menschlicher oder tierischer Körperstoffe, insbesondere Blut oder Gewebe, bei dem ein Trennverfahren (2) auf einer Unterlage (7) zur Vorbereitung einer nachfolgenden Analyse der Ausgangsprodukte des Trennverfahrens (2) eingesetzt wird, **dadurch gekennzeichnet, daß** die Probe (1) in einzelne Abschnitte (8) unterteilt und eine gezielte Analyse der Körperstoffe verschiedener Abschnitte (8) auf der Unterlage (7) durchgeführt wird.

2. Verfahren nach Anspruch 1, **dadurch gekennzeichnet, daß** die Analyse eines nachfolgenden Abschnittes (8) durch die erfaßten Merkmale des vorangegangenen Abschnittes (8) der Probe (1) bestimmt wird.

3. Verfahren nach den Ansprüchen 1 oder 2, **dadurch gekennzeichnet, daß** aufgrund der erfaßten Merkmale des vorangegangenen Abschnittes das Analyseverfahren (3) bestimmt wird.

4. Verfahren nach zumindest einem der vorhergehenden Ansprüche, **dadurch gekennzeichnet, daß** aufgrund des Ergebnisses der Analyse des vorangegangenen Abschnittes (8) der nachfolgend zu analysierende Abschnitt (8) ausgewählt wird.

5. Verfahren nach zumindest einem der vorhergehenden Ansprüche, **dadurch gekennzeichnet, daß** durch die Analyse zunächst eine Grobklassifikation vorgenommen wird, um so eine Eingrenzung der nachfolgend zu erfassenden Merkmale zu erreichen.

6. Verfahren nach zumindest einem der vorhergehenden Ansprüche, **dadurch gekennzeichnet, daß** mehrere Analyseverfahren (3) nacheinander durchgeführt werden, wobei die Auswahl oder die Reihenfolge durch das Ergebnis zumindest eines vorangegangenen Analyseverfahrens (3) bestimmt wird.

7. Verfahren nach zumindest einem der vorhergehenden Ansprüche, **dadurch gekennzeichnet, daß** die Abfolge der Analyse der verschiedenen Abschnitte (8) zusätzlich als Grundlage der Messerfassung hinzugezogen wird.

8. Vorrichtung (6) zur Erfassung von Merkmalen durch Analyse einer Probe (1) menschlicher oder tierischer Körperstoffe, insbesondere Blut oder Gewebe, mit einer die Körperstoffe tragenden Unterlage (7), **dadurch gekennzeichnet, daß** die Vorrichtung (1) zur ortsaufgelösten Erfassung von Meßwerten der auf der Unterlage (7) aufliegenden Körperstoffe ausgeführt ist.

9. Vorrichtung (6) nach Anspruch 8, **dadurch gekennzeichnet, daß** die Vorrichtung (6) zur optischen Merkmalserfassung mit einer Lichtquelle (11) und einem optischen Sensor (14) ausgeführt ist.

10. Vorrichtung (6) nach Anspruch 8 oder 9, **dadurch gekennzeichnet, daß** die Vorrichtung (6) zur Analyse verschiedener Abschnitte (8) der Probe (1) eine steuerbare Lichtablenkung aufweist.

11. Vorrichtung (6) nach zumindest einem der Ansprüche 8 bis 10, **dadurch gekennzeichnet, daß** die Unterlage (7) transmissiv ist.

12. Vorrichtung (6) nach zumindest einem der Ansprüche 8 bis 11, **dadurch gekennzeichnet, daß** die Vorrichtung (6) zur Analyse verschiedener Abschnitte (8) der Probe (1) mit einer relativ beweglichen Unterlage (7) ausgestattet ist.

13. Vorrichtung (6) nach zumindest einem der Ansprüche 8 bis 12, **dadurch gekennzeichnet, daß** die Vorrichtung (6) eine Ultraschallquelle und einen Ultraschallsensor aufweist.

14. Vorrichtung (6) nach zumindest einem der Ansprüche 8 bis 13, **dadurch gekennzeichnet, daß** die Vorrichtung (6) zusätzlich einen dem jeweiligen Abschnitt (8) der Unterlage (7) zuordnenbaren Sensor aufweist.

15. Vorrichtung (6) nach Anspruch 14, **dadurch gekennzeichnet, daß** der zusätzliche Sensor zur Erfassung der elektrischen Leitfähigkeit oder des PH-Wertes bestimmt ist.
